**Europäisches Patentamt**

**European Patent Office**

**Office européen des brevets**

(19)

(11) Veröffentlichungsnummer : **0 362 302 B1**

(12) **EUROPÄISCHE PATENTSCHRIFT**

(45) Veröffentlichungstag der Patentschrift :
**13.05.92 Patentblatt 92/20**

(51) Int. Cl.$^5$ : **A61K 7/13**

(21) Anmeldenummer : **89900603.5**

(22) Anmeldetag : **19.12.88**

(86) Internationale Anmeldenummer :
**PCT/EP88/01177**

(87) Internationale Veröffentlichungsnummer :
**WO 89/06531 27.07.89 Gazette 89/16**

(54) **MITTEL UND VERFAHREN ZUM OXIDATIVEN FÄRBEN, INSBESONDERE ZUM NACHFÄRBEN VON LEBENDEN HAAREN.**

(30) Priorität : **21.01.88 DE 3801606**

(43) Veröffentlichungstag der Anmeldung :
**11.04.90 Patentblatt 90/15**

(45) Bekanntmachung des Hinweises auf die
Patenterteilung :
**13.05.92 Patentblatt 92/20**

(84) Benannte Vertragsstaaten :
**FR GB NL**

(56) Entgegenhaltungen :
**EP-A-02 747 01
DE-A- 1 617 826
DE-A- 2 005 074
DE-A- 2 349 050
DE-A- 2 913 755
DE-A- 2 939 304**

(73) Patentinhaber : **GOLDWELL
AKTIENGESELLSCHAFT
Zerninstrasse 10-18
W-6100 Darmstadt 13 (DE)**

(72) Erfinder : **TENNIGKEIT, Jürgen
Felsbergstra e 51
W-6104 Seeheim 2 (DE)**
Erfinder : **LORENZ, Herbert
Ober Ramstädter Strasse 22
W-6101 Gross-Bieberau (DE)**
Erfinder : **SEGAWA, Hirotsugu
2-7-9, Nihonbashi
Minami-ku Osaka (JP)**

## Beschreibung

Die Erfindung betrifft ein Mittel für die oxidative Färbung von menschlichen oder tierischen Haaren, welches unmittelbar vor der Anwendung durch Vermischen einer, wenigstens einen Oxidationsfarbstoff enthaltenden creme-, gel- oder pulverförmigen Farbgrundlage und eines Oxidationsmittels hergestellt und auf einen pH-Wert im Bereich von 8,0 bis 10,5 eingestellt ist, sowie ein Verfahren zum Nachfärben von gefärbtem Haar, bei welchem aus einer, wenigstens einen Oxidationsfarbstoff enthaltenden creme-, gel- oder pulverförmigen Farbgrundlage und einem Oxidationsmittel ein in gebrauchsfertigem Zustand einen pH-Wert im Bereich zwischen 8,0 bis 10,5 aufweisendes Haarfärbemittel aufbereitet wird, welches dann auf den Nachwuchs der nachzufärbenden Haare aufgetragen und dort für eine vorgegebene Zeitdauer zur Einwirkung gebracht und anschließend in die bereits gefärbten Haarlängen und -spitzen durchgekämmt und dort eine weitere vorgegebene Zeitdauer zur Einwirkung gebracht und schließlich ausgewaschen wird.

Für die dauerhafte Färbung von menschlichen Haaren werden alkalische, wenigstens einen Oxidationsfarbstoff enthaltende Farbgrundlagen in Creme-, Gel- bzw. Pulverform verwendet, die unmittelbar vor der Anwendung mit einem sauren Oxidationsmittel, z.B. Wasserstoffperoxid bzw. einer Wasserstoffperoxid enthaltenden Emulsion zum gebrauchsfertigen, auf das zu färbende Haar aufzutragenden Haarfärbemittel vermischt werden. Die Färbung entsteht hierbei durch Reaktion bestimmter Entwicklersubstanzen mit bestimmten Kupplersubstanzen in Gegenwart eines geeigneten Oxidationsmittels. Das gebrauchsfertige Präparat liegt dabei deutlich im alkalischen Bereich, wodurch die Oberflächen der zu färbenden Haare in einer für das Eindringen der Oxidationsfarbstoffe vorteilhaften Weise aufgeschlossen werden. Andererseits beeinflussen alkalische Präparate die Haare auch, so daß es - insbesondere bei wiederholten Einwirkungen, z. B. bei mehrfachem Nachfärben - zu Schädigungen des Haares kommen kann. Solche Nachfärbungen sind aber erforderlich, um beispielsweise den ungefärbten Haarnachwuchs farblich an die bereits gefärbten Haarlängen anzugleichen, wobei dann in der Regel auch die früher gefärbten Haarlängen farblich aufgefrischt werden müssen, wenn sie im Laufe der Zeit durch äußere Einwirkungen, z.B. Sonnenbestrahlung, häufiges Waschen u. dgl., gegenüber der ursprünglichen Farbe aufgehellt sind.

DE-A-2 349 050 (Wella AG) beschreibt ein Verfahren und ein Mittel zur Herabsetzung der Wirkstoffkonzentration in alkalisch reagierenden kosmetischen Präparaten (u.a. Haarfärbemittel wie Oxidationsfarbstoffe). Kurz vor dem Gebrauch werden Verbindungen mit Ester und/oder Halogengruppen im Molekül eingesetzt (eventuell mikroverkapselt), die bei der Spaltung Säure entstehen lassen. Organische Säuren und der zu erreichende pH-Wert sind nicht erwähnt.

Der Erfindung liegt die Aufgabe zugrunde, ein Haarfärbemittel sowie ein Verfahren zur Aufbereitung und anschließenden Auftragung des Mittels auf das zu behandelnde Haar anzugeben, welche - auch bei mehrfacher Nachfärbung - nicht zu den geschilderten Haarschädigungen führen.

Das erfindungsgemäß zur Lösung dieser Aufgabe vorgesehene, aus einer creme-, gel- oder pulverförmigen, wenigstens einen Oxidationsfarbstoff enthaltenden Farbgrundlage und einem Oxidationsmittel aufbereitete Haarfärbemittel ist dadurch gekennzeichnet, daß es in frisch aufbereitetem Zustand eine mikroverkapselte, organische Säure enthält, wobei die Wandstärke der Mikrokapseln so eingestellt ist, daß sie innerhalb einer Zeitdauer zwischen 10 und 60 Minuten so weit gelöst oder geschwächt sind, daß die organische Säure alleine oder bei Ausübung einer zusätzlichen mechanischen Beanspruchung in das Färbemittel auszutreten vermag und dabei den pH-Wert des Färbemittels in den schwach alkalischen (pH $\leqq$ 8), neutralen oder sauren Bereich verschiebt.

Die organische Säure ist beispielsweise ein carboxylgruppenhaltiges Acrylharz (z.B. Rohagit S) oder Zitronensäure, Weinsäure oder Apfelsäure.

Von Vorteil ist auch die Verwendung bzw. die Mitverwendung von Ascorbinsäure als organische Säure, weil sie neben der - im Vergleich zu den vorgenannten organischen Säuren zeitlich verzögerten - Verschiebung des pH-Werts des Färbemittels im Sinne einer Neutralisation oder Säuerung zusätzlich auch reduzierend auf das im aufbereiteten Haarfärbemittel als Oxidationsmittel enthaltene Wasserstoffperoxid wirkt. Bei der reduzierenden Reaktion (Redox-Wirkung) entsteht exotherm Wärme, welche zu einer Beschleunigung des Färbevorgangs führt. Die Zugabe der verkapselten Ascorbinsäure bewirkt also zeitlich verzögert eine Verschiebung des pH-Wertes, eine Reduzierung des $H_2O_2$-Gehalts, eine Erwärmung der Farbmasse und damit eine Verkürzung der Einwirkzeit.

Beim Nachfärben von gefärbtem Haar wird in bekannter Weise so verfahren, daß aus einer, wenigstens einen Oxidationsfarbstoff enthaltenden creme-, gel- oder pulverförmigen Farbgrundlage und einem Oxidationsmittel ein in gebrauchsfertigem Zustand einen pH-Wert im Bereich zwischen 8,0 bis 10,5 aufweisendes Haarfärbemittel aufbereitet wird, welches dann auf den Nachwuchs der nachzufärbenden Haare aufgetragen und dort für eine vorgegebene Zeitdauer zur Einwirkung gebracht und anschließend in die bereits gefärbten Haarlängen und -spitzen durchgekämmt und dort eine weitere vorgegebene Zeitdauer zur Einwirkung gebracht und

2

schließlich ausgewaschen wird, wobei in erfindungsgemäßer Weiterbildung bei der Aufbereitung des Färbemittels eine organische Säure in mikroverkapselter Form in solcher Menge zugesetzt wird, daß der pH-Wert des Färbemittels bei Vermischung mit dieser organischen Säure gerade in den schwach alkalischen (pH $\leqq$ 8), neutralen bzw. leicht sauren Bereich verschoben wird, wobei die Wandstärke der im alkalischen Färbemittel löslichen oder in ihren Festigkeitseigenschaften im Sinne einer Schwächung veränderbaren Mikrokapseln so gewählt ist, daß die in den Mikrokapseln enthaltene sauer reagierende Verbindung innerhalb einer Zeitdauer zwischen 10 und 60 Minuten in das Färbemittel austritt. Das Durchkämmen des Färbemittels in die Haarlängen und -spitzen unterstützt dabei die Zerstörung von noch nicht vollständig gelösten oder der geschwächten Mikrokapseln sowie den Übertritt und die innige Vermischung der organischen Säure mit dem Färbemittel.

Bei Mitverwendung eines Indikatorfarbstoffs wird der Austritt in das bis dahin alkalische Färbemittel auch durch einen Farbumschlag angezeigt, welcher signalisiert, daß das bisher nur auf dem Nachwuchs aufgetragene Färbemittel im pH-Wert verringert ist und in die mittleren Haarlängen und die -spitzen gekämmt werden kann, um nunmehr in diesem Bereich in der bezüglich des pH-Wertes verringerten und somit keine weiteren Schädigungen auslösenden Form die Farbe aufzufrischen.

Die Aufbereitung des Haarfärbemittels kann dabei so erfolgen, daß die Farbgrundlage, das Oxidationsmittel und die mikroverkapselte organische Säure aus vor der Aufbereitung des gebrauchsfertigen Färbemittels voneinander getrennt gehaltenen Zusammensetzungen vermischt werden.

Alternativ können jedoch das Oxidationsmittel und die mikroverkapselte organische Säure auch im erforderlichen Mengenverhältnis vorgemischt mit der Farbgrundlage vermischt werden. Die Vormischung der mikroverkapselten organischen Säure mit dem Oxidationsmittel ist möglich, weil das saure Oxidationsmittel das Material der Mikrokapseln nicht angreift, so daß diese Vormischung also bereits beim Hersteller erfolgen und so in jedem Falle gewährleistet werden kann, daß das korrekte Mengenverhältnis von Oxidationsmittel und organischer Säure eingestellt wird. Anstelle einer der erwähnten organischen Säuren können auch zwei oder mehr dieser mikroverkapselten organischen Säuren verwendet werden, wobei diese dann entweder - sofern sie nicht miteinander reagieren - im gewählten Mengenverhältnis gemischt und dann gemeinsam oder auch getrennt mikroverkapselt sind.

Zur näheren Beschreibung der Erfindung seien die folgenden Beispiele angeführt:

## BEISPIEL 1

40 ml Färbepaste der unten angegebenen Rezeptur für die Farbe "Mittelblond" wurden mit 40 ml einer 6 %igen $H_2O_2$-Emulsion der ebenfalls unten angegebenen Rezeptur, die 7,5 % Citronensäure mikroverkapselt in Fumarsäure enthält, vermischt, woraus ein Haarfärbemittel mit einem pH-Wert von 9.5 resultierte. Das Mittel wurde auf den Nachwuchs eines bereits gefärbten, ca. 50 % ergrauten Haares aufgetragen und zur Einwirkung gebracht.

15 Minuten nach Beendigung des Auftragens hatten sich die Fumarsäure-Kapseln gelöst und der pH-Wert des Haarfärbemittels von 9.5 auf 7.0 verändert. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleichmäßiger Mittelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

Beispiel 1

## Rezeptur Färbepaste Mittelblond

| | |
|---|---|
| Cetylstearylalkohol | 10,00 g |
| Kokosfettsäuremonoäthanolamid | 2,00 g |
| Stearinsäuremonoäthanolamid | 2,00 g |
| Stearinsäurediäthanolamid | 1,00 g |
| p-Toluylendiaminsulfat | 0,50 g |
| Resorcin | 0,10 g |
| 4-Chlorresorcin | 0,10 g |
| m-Aminophenol | 0,02 g |
| Monoäthanolamin | 7,00 g |
| Ammoniumchlorid | 0,50 g |
| Parfüm | 0,30 g |

Enth. Wasser      auf      100,00 g

## Rezeptur $H_2O_2$-Emulsion

| | |
|---|---|
| Hydroxyäthylcellulose | 0,50 g |
| o-Phosphorsäure 85 % | 0,19 g |
| Wasserstoffpeoxid 50 %, stabilisiert | 12,00 g |
| In Fumarsäure mikroverkapselte | |
| Citronensäure | 7,5 g |
| Enth. Wasser    auf | 100,00 g |

**BEISPIEL 2**

40 ml Färbepaste der unten angegebenen Rezeptur für die Farbe "Mittelblond" wurden mit 40 ml einer 6 %igen $H_2O_2$-Emulsion der ebenfalls unten angegebenen Rezeptur, die 17,5 % carboxylgruppenhaltiges Acrylharz (Rohagit S env) mikroverkapselt in Celluloseacetatphthalat enthält, vermischt, woraus ein Haarfärbemittel mit einem pH-Wert von 9.5 resultierte. Das Mittel wurde auf den Nachwuchs eines bereits gefärbten, ca. 50 % ergrauten Haares aufgetragen und zur Einwirkung gebracht.

15 Minuten nach Beendigung des Auftragens hatten sich die Celluloseacetatphthalat-Kapseln gelöst und der pH-Wert des Haarfärbemittels von 9.5 auf 7.0 verändert, was auch optisch durch eine Dunkelfärbung der Farbmasse angezeigt wurde. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleich mäßiger Mittelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

**Beispiel 2**

**Rezeptur Färbepaste Mittelblond**

| | |
|---|---|
| Cetylstearylalkohol | 10,00 g |
| Kokosfettsäuremonoäthanolamid | 2,00 g |
| Stearinsäuremonöathanolamid | 2,00 g |
| Stearinsäurediäthanolamid | 1,00 g |
| p-Toluylendiaminsulfat | 0,50 g |
| Resorcin | 0,10 g |
| 4-Chlorresorcin | 0,10 g |
| m-Aminophenol | 0,02 g |
| Monoäthanolamin | 7,00 g |
| Ammoniumchlorid | 0,50 g |
| Parfüm | 0,30 g |
| Enth. Wasser    auf | 100,00 g |

4

Rezeptur H$_2$O$_2$-Emulsion

| | |
|---|---|
| Hydroxyäthylcellulose | 0,50 g |
| o-Phosphorsäure 85 % | 0,19 g |
| Wasserstoffpeoxid 50 %, stabilitiert | 12,00 g |
| In Celluloseacetatphthalat mikroverkapseltes carboxylgruppenhaltiges Acrylharz (Rohagit S) | 17,50 g |
| Enth. Wasser auf | 100,00 g |

**BEISPIEL 3**

40 ml Färbepaste der unten angegebenen Rezeptur für die Farbe "Haselnußblond" wurden mit 40 ml einer 6 %igen H$_2$O$_2$-Emulsion der ebenfalls unten angegebenen Rezeptur, die 7,5 % Citronensäure mikroverkapselt in Fumarsäure enthält, vermischt, woraus ein Haarfärbemittel mit einem pH-Wert von 9.5 resultierte. Das Mittel wurde auf den Nachwuchs eines bereits gefärbten, ca. 30 % ergrauten Haares aufgetragen und zur Einwirkung gebracht.

15 Minuten nach Beendigung des Auftragens hatten sich die Fumarsäure-Kapseln gelöst und der pH-Wert des Haarfärbemittels von 9.5 auf 7.0 verändert. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleichmäßiger Haselnußblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

**Biespiel 3**

Rezeptur Färbepaste Haselnußblond

| | |
|---|---|
| Cetylstearylalkohol | 10,00 g |
| Kokosfettsäuremonoäthanolamid | 2,00 g |
| Stearinsäuremonoäthanolamid | 2,00 g |
| Stearinsäurediäthanolamid | 1,00 g |
| p-Toluylendiaminsulfat | 0,45 g |
| Resorcin | 0,10 g |
| m-Aminophenol | 0,05 g |
| 4-Nitro-2-aminophenol | 0,05 g |
| p-Aminophenol | 0,30 g |
| p-Amino-o-kresol | 0,05 g |
| 2-Methylresorcin | 0,10 g |
| Ammoniak 25 % | 7,50 g |
| Natriumsulfit | 1,00 g |
| Äthylendiamintetraessigsäure | 0,60 g |
| Ammoniumchlorid | 0,50 g |
| Parfüm | 0,30 g |
| Enth. Wasser auf | 100,00 g |

## Rezeptur H$_2$O$_2$-Emulsion

| | |
|---|---|
| Hydroxyäthylcellulose | 0,50 g |
| o-Phosphorsäure 85 % | 0,19 g |
| Wasserstoffpeoxid 50 %, stabilisiert | 12,00 g |
| In Fumarsäure mikroverkapselte Citronensäure | 7,5 g |
| Enth. Wasser auf | 100,00 g |

**BEISPIEL 4**

40 ml Färbepaste der unten angegebenen Rezeptur für die Farbe "Haselnußblond" wurden mit 40 ml einer 6 %igen H$_2$O$_2$-Emulsion der ebenfalls unten angegebenen Rezeptur, die 17,5 % carboxylgruppenhaltiges Acrylharz (Rohagit S env) mikroverkapselt in Celluloseacetatphthalat enthält, vermischt, woraus ein Haarfärbemittel mit einem pH-Wert von 9.5 resultierte. Das Mittel wurde auf den Nachwuchs eines bereits gefärbten, ca. 30 % ergrauten Haares aufgetragen und zur Einwirkung gebracht.

15 Minuten nach Beendigung des Auftragens hatten sich die Celluloseacetatphthalat-Kapseln gelöst und der pH-Wert des Haarfärbemittels von 9.5 auf 7.0 verändert, was auch optisch durch eine Dunkelfärbung der Farbmasse angezeigt wurde. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleichmäßiger Haselnußblond-Farbton erhalten.

Das graue Haar am Ansatz war vollständig abgedeckt, Mittelstück und Spitzen der Haare wirkten gepflegt, was sich durch Glanz und weichen Griff bemerkbarmachte.

Beispiel 4

**Rezeptur Färbepaste Haselnußblond**

| | |
|---|---|
| Cetylstearylalkohol | 10,00 g |
| Kokosfettsäuremonoäthanolamid | 2,00 g |
| Stearinsäuremonoäthanolamid | 2,00 g |
| Stearinsäurediäthanolamid | 1,00 g |
| p-Toluylendiaminsulfat | 0,45 g |
| Resorcin | 0,10 g |
| m-Aminophenol | 0,05 g |
| 4-Nitro-2-aminophenol | 0,05 g |
| p-Aminophenol | 0,30 g |
| p-Amino-o-kresol | 0,05 g |
| 2-Methylresorcin | 0,10 g |
| Ammoniak 25 % | 7,50 g |
| Natriumsulfit | 1,00 g |
| Äthylendiamintetraessigsäure | 0,60 g |
| Ammoniumchlorid | 0,50 g |
| Parfüm | 0,30 g |
| Enth. Wasser auf | 100,00 g |

**Rezeptur H$_2$O$_2$-Emulsion**

| | |
|---|---|
| Hydroxyäthylcellulose | 0,50 g |
| o-Phosphorsäure 85 % | 0,19 g |
| Wasserstoffpeoxid 50 %, stabilitiert | 12,00 g |
| In Celluloseacetatphthalat mikroverkapseltes carboxylgruppenhaltiges Acrylharz (Rohagit S) | 17,50 g |
| Enth. Wasser                   auf | 100,00 g |

**BEISPIEL 5**

20 g Färbepulver der unten angegebenen Rezeptur für die Farbe "Dunkelblond" wurden mit 40 ml einer 6 %igen H$_2$O$_2$-Emulsion der ebenfalls unten angegebenen Rezeptur, die 7,5 % Citronensäure mikroverkapselt in Fumarsäure enthält, vermischt, woraus ein Haarfärbemittel mit einem pH-Wert von 9.5 resultierte. Das Mittel wurde auf den Nachwuchs eines bereits gefärbten, ca. 30 % ergrauten Haares aufgetragen und zur Einwirkung gebracht.

15 Minuten nach Beendigung des Auftragens hatten sich die Fumarsäure-Kapseln gelöst und der pH-Wert des Haarfärbemittels von 9.5 auf 7.0 verändert, was auch optisch durch eine Dunkelfärbung der Farbmasse angezeigt wurde. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleichmäßiger Dunkelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

**Beispiel 5**

**Rezeptur Färbepulver Dunkelblond**

| | |
|---|---|
| Hydroxy-Äthylcellulose | 2,00 g |
| Natriummetasilikat | 6,00 g |
| Siliciumdioxid (Aerosil) | 90,10 g |
| Äthylendiamintetraessigsäure | 0,60 g |
| Ammoniumchlorid | 0,50 g |
| p-Toluylendiaminsulfat in Gelatine mikroverkapselt | 0,50 g |
| Resorcin | 0,20 g |
| m-Aminophenol in Gelatine mikroverkapselt | 0,10 g |
| | 100,00 g |

### Rezeptur H₂O₂-Emulsion

| | | |
|---|---|---|
| Hydroxyäthylcellulose | | 0,50 g |
| o-Phosphorsäure 85 % | | 0,19 g |
| Wasserstoffpeoxid 50 %, stabilisiert | | 12,00 g |
| In Fumarsäure mikroverkapselte | | |
| Citronensäure | | 7,50 g |
| Enth. Wasser | auf | 100,00 g |

**BEISPIEL 6**

20 g Färbepulver der unten angegebenen Rezeptur für die Farbe "Dunkelblond" wurden mit 40 ml Wasser vermischt, woraus durch die sofortige Auflösung der Gelatine-Kapseln ein Haarfärbemittel mit einem pH-Wert von 9.5 resultierte. Das Mittel wurde auf den Nachwuchs eines bereits gefärbten, ca. 30 % ergrauten Haares aufgetragen und zur Einwirkung gebracht.

15 Minuten nach Beendigung des Auftragens hatten sich die Fumarsäure-Kapseln gelöst und der pH-Wert des Haarfärbemittels von 9.5 auf 7.0 verändert, was auch optisch durch eine Dunkelfärbung der Farbmasse angezeigt wurde. Nach diesen 15 Minuten wird die jetzt neutral bis schwach sauer reagierende Farbmasse in die Haarlängen und Spitzen gekämmt.

Die Farbmasse wurde hier 10 - 15 Minuten einwirken lassen und anschließend ausshampooniert.

Als Ergebnis wurde ein gleichmäßiger Dunkelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

**Biespiel 6**

### Rezeptur Färbepulver Dunkelblond

| | |
|---|---|
| Hydroxyäthylcellulose | 2,00 g |
| Natriummetasilikat | 6,00 g |
| Siliciumdioxid (Aerosil) | 72,60 g |
| Äthylendiamintetraessigsäure | 0,60 g |
| Ammoniumchlorid | 0,50 g |
| Resorcin | 0,20 g |
| p-Toluylendiaminsulfat in Gelatine mikroverkapselt | 0,50 g |
| m-Aminophenol in Gelatine mikroverkapselt | 0,10 g |
| Harnstoffperoxid in Gelatine mikroverkapselt | 10,00 g |
| Citronensäure in Fumarsäure mikroverkapselt | 7,50 g |
| | 100,00 g |

**BEISPIEL 7**

40 ml Färbepaste der unten angegebenen Rezeptur für die Farbe "Mittelblond" wurden mit 40 ml einer 6 %igen $H_2O_2$-Emulsion der ebenfalls unten angegebenen Rezeptur vermischt, woraus ein Haarfärbemittel mit einem pH-Wert von 9.5 resultierte. Dazu wurden 3,2 g einer mikroverkapselten Citronensäure, deren Wandmaterial aus Copolymerisaten von Acryl- und Methacrylsäureestern wie z.B. Eudragit L30D und Eudragit RS30D bestand, beigemischt. Das Gewichtsverhältnis Wandmaterial/Kernmaterial wurde mit 30 zu 70 gewählt. Das Mittel wurde auf den Nachwuchs eines bereits gefärbten, ca. 50 % ergrauten Haares aufgetragen und zur Einwirkung gebracht.

15 Minuten nach beendigung des Auftragens wurde die gesamte Farbmasse in die Haarlängen und Spitzen gekämmt. Durch die mechanische Reibung des Kämmens wurden die in der Farbmasse weich gewordenen Kapselwandungen zerstört und der pH-Wert des Haarfärbemittels durch die austretende Citronensäure von pH 9.5 auf pH 6.8 verändert.

Die Farbmasse wurde hier 15 Minuten einwirken lassen und anschließend ausshampooniert.

Als Ergebnis wurde ein gleichmäßiger Mittelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.


Beispiel 7


**Rezeptur Färbepaste Mittelblond**

| | |
|---|---|
| Cetylstearylalkohol | 10,00 g |
| Kokosfettsäuremonoäthanolamid | 2,00 g |
| Stearinsäuremonoäthanolamid | 2,00 g |
| Stearinsäurediäthanolamid | 1,00 g |
| p-Toluylendiaminsulfat | 0,50 g |
| Resorcin | 0,10 g |
| 4-Chlorresorcin | 0,10 g |
| m-Aminophenol | 0,02 g |
| Monoäthanolamin | 7,00 g |
| Ammoniumchlorid | 0,50 g |
| Parfüm | 0,30 g |
| Enth. Wasser                auf | 100,00 g |


**Rezeptur $H_2O_2$-Emulsion**

| | |
|---|---|
| Hydroxyäthylcellulose | 0,50 g |
| o-Phosphorsäure 85 % | 0,19 g |
| Wasserstoffpeoxid 50 %, stabilisiert | 12,00 g |
| Enth. Wasser                auf | 100,00 g |


**BEISPIEL 8**

40 ml Färbepaste der unten angegebenen Rezeptur für die Farbe "Mittelblond" wurden mit 40 ml einer 6 %igen $H_2O_2$-Emulsion der ebenfalls unten angegebenen Rezeptur vermischt, woraus ein Haarfärbemittel mit einem pH-Wert von 9.5 resultierte. Dazu wurden 3,2 g einer mikroverkapselten Ascorbinsäure, deren Wandmaterial aus Copolymerisaten von Acryl- und Methacrylsäureestern wie z.B. Eudragit L30D und Eudragit RS30D bestand beigemischt. Der Anteil des Wandmaterials betrug dabei 30 Gew. %. Das Mittel wurde auf den Nachwuchs eines bereits gefärbten, ca. 50 % ergrauten Haares aufgetragen und zur Einwirkung gebracht.

15 Minuten nach Beendigung des Auftragens wurde die gesamte Farbmasse in die Haarlängen und Spitzen gekämmt. Durch die mechanische Reibung des Kämmens wurden die in der Farbmasse weich gewordenen Kapselwandungen zerstört. Der pH-Wert des Haarfärbemittels änderte sich durch die austretende Ascorbinsäure von pH 9.5 auf pH 7.1, die Temperatur der Farbmasse stieg von 25° C auf 38° C und der $H_2O_2$-Gehalt

der Farbmasse fiel von 2,92 % auf 1,50 %.

Die Farbmasse wurde hier 8 Minuten einwirken lassen und anschließend ausshampooniert.

Als Ergebnis wurde ein gleichmäßiger Mittelblond-Farbton erhalten.

Das graue Haar am Ansatz war vollständig abgedeckt, Mittelstück und Spitzen der Haare wirkten gepflegt, was sich durch Glanz und weichen Griff bemerkbar machte.

Beispiel 8

**Rezeptur Färbepaste Mittelblond**

| | |
|---|---|
| Cetylstearylalkohol | 10,00 g |
| Kokosfettsäuremonoäthanolamid | 2,00 g |
| Stearinsäuremonoäthanolamid | 2,00 g |
| Stearinsäurediäthanolamid | 1,00 g |
| p-Toluylendiaminsulfat | 0,50 g |
| Resorcin | 0,10 g |
| 4-Chlorresorcin | 0,10 g |
| m-Aminophenol | 0,02 g |
| Monoäthanolamin | 7,00 g |
| Ammoniumchlorid | 0,50 g |
| Parfüm | 0,30 g |
| Enth. Wasser auf | 100,00 g |

**Rezeptur $H_2O_2$-Emulsion**

| | |
|---|---|
| Hydroxyäthylcellulose | 0,50 g |
| o-Phosphorsäure 85 % | 0,19 g |
| Wasserstoffpeoxid 50 %, stabilisiert | 12,00 g |
| Enth. Wasser auf | 100,00 g |

**Patentansprüche**

1. Mittel für die oxidative Färbung von menschlichen oder tierischen Haaren, welches unmittelbar vor der Anwendung durch Vermischen einer, wenigstens einen Oxidationsfarbstoff enthaltenden creme-, gel- oder pulverförmigen Farbgrundlage und eines Oxidationsmittels hergestellt und auf einen pH-Wert im Bereich von 8,0 bis 10,5 eingestellt ist, **dadurch gekennzeichnet,** daß das Mittel in frisch aufbereitetem Zustand eine mikroverkapselte organische Säure enthält, wobei die Wandstärke der Mikrokapseln so eingestellt ist, daß sie innerhalb einer Zeitdauer zwischen 10 und 60 Minuten soweit gelöst oder geschwächt sind, daß die organische Säure alleine oder bei Ausübung einer zusätzlichen mechanischen Beanspruchung in das Färbemittel auszutreten vermag und dabei den pH-Wert des Färbemittels in den schwach alkalischen (pH $\leqq$ 8), neutralen oder sauren Bereich verschiebt.

2. Mittel nach Anspruch 1, dadurch gekennzeichnet, daß die organische Säure Zitronensäure, Weinsäure, Ascorbinsäure und/oder Apfelsäure ist.

3. Mittel nach Anspruch 1, dadurch gekennzeichnet, daß die organische Säure ein carboxylhaltiges Acrylharz ist.

4. Mittel nach einem der Ansprüche 2 bis 3, dadurch gekennzeichnet, daß in der mikroverkapselten organischen Säure ein bei Vermischung mit dem alkalischen Färbemittel einen Farbumschlag auslösender Indikatorstoff enthalten ist.

5. Verfahren zum Nachfärben von gefärbtem Haar, bei welchem aus einer wenigstens einen Oxidationsfarbstoff enthaltenden creme-, gel- oder pulverförmigen Farbgrundlage und einem Oxidationsmittel ein in gebrauchsfertigem Zustand einen pH-Wert im Bereich zwischen 8,0 bis 10,5 aufweisendes Haarfärbemittel aufbereitet wird, welches dann auf den Nachwuchs der nachzufärbenden Haare aufgetragen und dort für eine vorgegebene Zeitdauer zur Einwirkung gebracht und anschließend in die bereits gefärbten Haarlängen und -spitzen durchgekämmt und dort eine weitere vorgegebene Zeitdauer zur Einwirkung gebracht und schließlich ausgewaschen wird, dadurch gekennzeichnet, daß bei der Aufbereitung des Färbemittels eine mikroverkapselte organische Säure in solcher Menge zugesetzt wird, daß der pH-Wert des Färbemittels bei Vermischung mit dieser in den schwach alkalischen (pH $\leqq$ 8), neutralen bzw. sauren Bereich verschoben wird, wobei die Wandstärke der im alkalischen Färbemittel löslichen oder in ihren Festigkeitseigenschaften im Sinne einer Schwächung veränderbaren Mikrokapseln so gewählt ist, daß die in den Mikrokapseln enthaltene organische Säure innerhalb einer Zeitdauer zwischen 10 und 60 Minuten in das Färbemittel austritt und die Verringerung des pH-Werts bewirkt.

6. Verfahren nach Anspruch 5, dadurch gekennzeichnet, daß die wenigstens einen Oxidationsfarbstoff enthaltende Farbgrundlage, das Oxidationsmittel und die mikroverkapselte organische Säure aus vor der Aufbereitung des gebrauchsfertigen Färbemittels voneinander getrennt gehaltenen Vorratsmengen zusammengemischt werden.

7. Verfahren nach Anspruch 6, dadurch gekennzeichnet, daß das Oxidationsmittel und die mikroverkapselte organische Säure im erforderlichen Mengenverhältnis vorgemischt mit der Farbgrundlage vermischt werden.

## Claims

1. Composition for the oxidative dyeing of human or animal hair, which is prepared immediately before application by mixing an oxidising agent and a cream, gel or powder dye base containing at least one oxidative dye, and which is adjusted to a pH value in the range of from 8.0 to 10.5,
characterised in that
the composition in the freshly prepared condition contains a microencapsulated organic acid, the wall thickness of the microcapsules being such that, within a period of from 10 to 60 minutes, they are dissolved or weakened to such an extent that the organic acid is able to pass into the dyeing composition on its own or on exertion of an additional mechanical load and in so doing adjusts the pH value of the dyeing composition into the weakly alkaline (pH $\leqq$ 8), neutral or acidic range.

2. Composition according to claim 1, characterised in that the organic acid is citric acid, tartaric acid, ascorbic acid and/or malic acid.

3. Composition according to claim 1, characterised in that the organic acid is a carboxyl-containing acrylic resin.

4. Composition according to either claim 2 or claim 3, characterised in that the microencapsulated organic acid contains an indicator which brings about a colour change upon mixing with the alkaline dyeing composition.

5. Process for re-dying dyed hair, in which a hair-dyeing composition having a pH value in the range of from 8.0 to 10.5 in the ready-for-use condition is prepared from an oxidising agent and a cream, gel or powder dye base containing at least one oxidative dye, which hair-dyeing composition is then applied to the recent growth of the hair to be re-dyed and is allowed to act there for a given period of time and is then combed through the already dyed hair to the ends and is allowed to act there for a further given period of time, and finally is washed out, characterised in that, when the dyeing composition is prepared, a microencapsulated organic acid is added in such an amount that the pH value of the dyeing composition is adjusted into the weakly alkaline (pH $\leqq$ 8), neutral or acidic range when the dyeing composition mixes with the organic acid, the wall thickness of the microcapsules, which are soluble in the alkaline dyeing composition or whose strength properties can be so altered that they become weakened, being so selected that the organic acid contained in the microcapsules passes into the dyeing composition within a period of from 10 to 60 minutes and brings about the decrease in the pH value.

6. Process according to claim 5, characterised in that the dye base containing at least one oxidative dye, the oxidising agent and the microencapsulated organic acid are mixed together from stocks which are kept separate prior to preparation of the ready-for-use dyeing composition.

7. Process according to claim 6, characterised in that the oxidising agent and the microencapsulated organic acid are premixed in the required ratio before being mixed with the dye base.

## Revendications

1. Agent de coloration oxydante de cheveux humains ou animaux qui est fabriqué peu avant son application par le mélange d'une base de couleur en forme de crème, de gel ou de poudre, contenant au moins un colorant d'oxydation, et d'un produit oxydant et qui est ajusté à une valeur du pH située entre 8,0 et 10,5,
caractérisé en ce que
l'agent contient un acide organique microencapsulé à l'état de préparation frais, les microcapsules ayant une résistance de paroi choisie de manière qu'après une durée de 10 à 60 minutes ces parois soient dissoutes ou affaiblies au point que l'acide organique est en mesure de s'écouler dans le colorant de lui-même, ou par l'action d'une contrainte mécanique supplémentaire et déplace ainsi la valeur du pH du colorant vers l'état faiblement alcalin (pH $\leqq$ 8), neutre ou acide.

2. Agent selon la revendication 1, caractérisé en ce que l'acide organique est de l'acide citrique, de l'acide tartrique, de l'acide ascorbique et/ou de l'acide malique.

3. Agent selon la revendication 1, caractérisé en ce que l'acide organique est une résine acrylique contenant des groupes carboxyles.

4. Agent selon l'une des revendications 2 ou 3, caractérisé en ce que l'acide organique microencapsulé contient un indicateur déclenchant un virage de teinte lors du mélange avec le colorant alcalin.

5. Procédé de recoloration de cheveux colorés, du type selon lequel un colorant de cheveux ayant, quand il est prêt à l'emploi, une valeur du pH entre 8,0 et 10,5 est préparé à partir d'une base de couleur sous forme de crème, de gel ou de poudre, contenant au moins un colorant d'oxydation, ainsi que d'un agent oxydant, qui est alors appliqué sur les nouvelles pousses de cheveux à recolorer et laissé agir là pendant une durée prédéterminée, puis le colorant est entraîné par peignage sur les longueurs et les pointes de cheveux déjà colorées et laissé agir là pendant une autre durée prédéterminée, et finalement entraîné par lavage, caractérisé en ce qu'un acide organique microencapsulé est ajouté dans la préparation de colorant en une quantité telle que la valeur du Ph du colorant soit déplacée vers un état faiblement alcalin (pH $\leqq$ 8), neutre et/ou acide lorsqu'il est mélangé avec l'acide, la résistance de paroi des microcapsules solubles dans le colorant alcalin ou dont les propriétés de résistance se modifient dans le sens d'un affaiblissement, étant choisie de telle sorte que l'acide organique contenu dans les microcapsules s'écoule dans le colorant au cours d'une période de 10 à 60 minutes et provoque une diminution de la valeur du pH.

6. Procédé selon la revendication 5, caractérisé en ce que la base de couleur contenant au moins un colorant d'oxydation, l'agent oxydant et l'acide organique microencapsulé sont mélangés ensemble à partir de quantités conservées séparément les unes des autres avant la préparation du colorant prêt à l'emploi.

7. Procédé selon la revendication 6, caractérisé en ce que l'agent oxydant et l'acide organique microencapsulé, prémélangés dans le rapport nécessaire sont mélangés avec la base de couleur.